# EUROPÄISCHE PATENTSCHRIFT

(11) **EP 0 813 057 B1**
(45) Veröffentlichungstag und Bekanntmachung des Hinweises auf die Patenterteilung: **11.03.2009**
(21) Anmeldenummer: 97106854.9
(22) Anmeldetag: 25.04.1997
(51) Int. Cl.: G01N 27/411

(54) **Vorrichtung zur Durchführung von elektrochemischen Messungen in Glas- oder Salzschmelzen**
Device for carrying out electrochemical measurements in glass or salt melts
Dispositif de réalisation de mesures électrochimiques dans des fusions de verre ou de sel

(30) Priorität: 14.06.1996 DE 19623683
(43) Veröffentlichungstag der Anmeldung: 17.12.1997
(73) Patentinhaber: Heraeus Electro-Nite International N.V., 3530 Houthalen (BE)
(72) Erfinder: Plessers, Jacques Josef, Dr., 3530 Houthalen (BE); Straetemans, Marc, 3941 Eksel (BE)
(74) Vertreter: Kühn, Hans-Christian

(56) Entgegenhaltungen:
- DE-A- 2 001 012
- DE-A- 3 303 851
- FR-A- 2 122 758
- GB-A- 2 057 695
- US-A- 3 625 026
- US-A- 3 816 269
- US-A- 4 313 799
- J. ELECTROCHEM. SOC., Bd. 119, Nr. 2, Februar 1972, Seiten 198-208, XP002064944
- MÜLLER-SIMON H. ET AL: 'Sensor for oxygen activity measurements in glass melts' GLASTECHNISCHE BERICHTE Bd. 64, Nr. 2, 01 Februar 1991, FRANKFURT,

## Beschreibung

Die Erfindung betrifft eine Vorrichtung zur Durchführung von elektrochemischen Messungen in Glas- oder Salzschmelzen mit wenigstens einer Meßelektrode und einer Referenzelektrodenanordnung.

Derartige Vorrichtungen sind vielfach, beispielsweise aus GB 2 057 695 A bekannt. Hier erfolgt eine Messung des Sauerstoffpartialdruckes mittels einer elektrochemischen Meßzelle, auch Referenzelektrodenanordnung genannt, die über eine übliche Anzeige- und/oder Auswerteeinrichtung (Meßsystem) mit einer Gegenelektrode (auch Meßelektrode) verbunden ist. Als Meßelektrode wird ein Platindraht verwendet, der durch einen Aluminiumoxid-Körper hindurchgeführt ist. An der Spitze des Aluminiumoxid-Körpers ist der Platindraht freiliegend, so daß er in Kontakt mit der Schmelze treten kann, sobald die Gegenelektrode in diese eintaucht. Der Auminiumoxid-Körper ist in einem Aluminiumoxidrohr gehaltert. In der Praxis hat es sich gezeigt, daß es nicht möglich ist, eine gasdichte Durchführung zwischen der Platinelektrode und dem Aluminiumoxid-Körper zu schaffen. Dadurch dringt Sauerstoff aus der Atmosphäre oberhalb der Schmelze bis zu dem mit der Schmelze in Kontakt stehenden Teil der Meßelektrode, so daß die dort gemessenen Werte nicht den tatsächlichen Verhältnissen innerhalb der Schmelze entsprechen und die Messung dadurch fehlerbehaftet ist.

Ähnliche Meßanordnungen sind beispielsweise auch aus DE 38 11 915 A1 bekannt. Auch hier ist die Meßelektrode aus Platin gebildet.

Beispielsweise aus "Glastechnische Berichte" 68 (1995) No. 9, S. 273 ff. ist es bekannt, durch voltametrische Analyse mit drei Elektroden Eisen, Schwefel oder Chrom in Glasschmelzen zu bestimmen. Auch hier treten die genannten Probleme auf. So muß zum Beispiel die Größe der Elektrodenoberfläche im Glas genau bekannt sein.

DE 2 001 012 offenbart ein elektrochemisches Verfahren zur Bestimmung von metallischen Calcium in Blei-Calciumlegierungen unter Verwendung von Iridium-Elektrodenspitzen.

Aufgabe der vorliegenden Erfindung ist es, ausgehend von den aus dem Stand der Technik bekannten Vorrichtungen die Meßgenauigkeit von beispielsweise Sauerstoffpartialdruckmessungen in Glas- oder Salzschmelzen zu verbessern.

Erfindungsgemäß wird die Aufgabe dadurch gelöst, daß die zum Eintauchen in die Schmelze bestimmte Spitze der Meßelektrode aus Iridium oder einer Iridiumlegierung mit anderen Edelmetallen gebildet ist und in einem hitzebeständigen Rohr gasdicht gehaltert ist, wobei die Meßelektrode durch das hitzebeständige Rohr hindurchgeführt ist (aus der Schmelze heraus zur Auswerteeinrichtung). Eine gasdichte Halterung bedeutet, daß kein Sauerstoff durch das Rohr von außen bis zur zu messenden Schmelze (insbesondere einer Glasschmelze) in einer solchen Menge dringt, die die Messung beeinflußt. Iridium oder Iridiumlegierungen sind hochschmelzend und können deshalb durch Wärmebehandlung mit dem hitzebeständigen Rohr gasdicht verbunden werden. Vorteilhafterweise kann das hitzebeständige Rohr ein Quarzglasrohr sein, es sind jedoch auch Keramikrohre, beispielsweise aus Aluminiumoxid möglich. Bei Verwendung eines Keramikrohres ist es natürlich notwendig, daß das Material bei Temperaturen von etwa 1000 bis 1500° C kein Ionen- oder Elektronenleiter ist.

Zweckmäßigerweise ist das Iridium- oder die Iridiumlegierung in das hitzebeständige Rohr gesintert oder eingeschmolzen. Iridium hat einen Schmelzpunkt von 2447° C, es hält daher der Erhitzung, wie sie zum Schmelzen oder dichten Sintern von Aluminiumoxid oder zum Erweichen von Quarzglas notwendig ist, stand.

Es ist denkbar, die Meßelektrode im Falle eines Einschmelzens in ein Quarzglasrohr überwiegend für Kurzzeitmessungen einzusetzen, während in Aluminiumoxidrohre eingeschmolzene Meßelektroden auch für Langzeitmessungen (mit sogenannten kontinuierlichen Sonden) eingesetzt werden können.

Zweckmäßig ist es, daß die Verbindung zwischen Meßelektrode und hitzebeständigem Rohr an der zum Eintauchen in die Schmelze bestimmten Spitze des Rohres gasdicht ist. Nach hinten, aus der Schmelze heraus, kann das Rohr offen sein. Zweckmäßig ist es, daß die aus Iridium oder einer Iridiumlegierung gebildete Spitze der Meßelektrode innerhalb des Rohres mit einem Meßdraht verbunden ist, der vorzugsweise aus Molybdän, Wolfram oder einer Chrom-Nickel-Legierungen (z. B. Cronix) gebildet ist. Dadurch kann die Länge des als Elektrode verwendeten Iridium-Drahtes kurz gehalten werden, um Iridium einzusparen.

Im Falle der Ausbildung des Meßdrahtes aus Molybdän oder Wolfram ist es möglich, zwischen der aus Iridium oder einer Iridiumlegierung gebildeten Spitze der Meßelektrode und dem Meßdraht einen Metallstreifen aus Molybdän anzuordnen. Die Verbindung zwischen Iridium und Meßdraht kann in dem hitzebeständigen Rohr eingeschmolzen sein. Insbesondere ein eingeschmolzener Metallstreifen aus Molybdän sichert eine nahezu absolute Gasdichtheit.

Ein Chrom-Nickel-Draht kann nicht ohne weiteres in das hitzebeständige Rohr eingeschmolzen werden, da die Gefahr besteht, daß es bei der notwendigen Temperatur schmilzt; die Verbindungsstelle ist daher vorzugsweise hinter der Einschmelzstelle in dem Rohr anzuordnen.

Vorteilhaft für eine hohe Meßgenauigkeit ist es, daß eine Referenzelektrode in einem einseitig geschlossenen Festelektrolytröhrchen angeordnet ist, das mit seinem dem geschlossenen Ende abgewandten Ende in einem Keramikrohr gehaltert ist, durch das die Referenzelektrode hindurchgeführt ist und daß das Ende der Referenzelektrode in dem Festelektrolytröhrchen von einem Referenzmaterial umgeben ist, daß aus einer Metall-Metalloxid-, vorzugsweise einer Nickel-Nickel-Oxid-Pulvermischung gebildet ist; die Referenzelektrode selbst ist zweckmäßigerweise aus einer Chrom-Nickel-Legierung gebildet.

Zweckmäßig ist es weiterhin, daß das hitzebeständige Rohr und das Keramikrohr mit Korund gefüllt sind. Desweiteren ist es vorteilhaft, daß das hitzebeständige Rohr und das Keramikrohr in einem gemeinsamen Trägerrohr gehaltert sind, das vorzugsweise aus Keramik gebildet ist und das an seinem dem Eintauchende abgewandten Ende ein Verbindungsstück üblicher Art aufweist zur mechanischen Ankopplung und zur Verbindung von Meßelektrode und Referenzelektrode mit einem Meßsystem. Das Trägerrohr kann aus Aluminiumoxid gebildet und mit Kugelkorund gefüllt sein.

Nachfolgend wird ein Ausführungsbeispiel der Erfindung anhand einer Zeichnung näher erläutert.

In der Zeichnung zeigt
- Figur 1: eine Darstellung der erfindungsgemäßen Vorrichtung,
- Figur 2: einen Längsschnitt durch die erfindungsgemäße Vorrichtung,
- Figur 3: einen Längsschnitt durch die in das hitzebeständigen Rohr eingeschmolzene Meßelektrode und
- Figur 4: einen Schnitt durch eine Vorrichtung mit drei Elektroden.

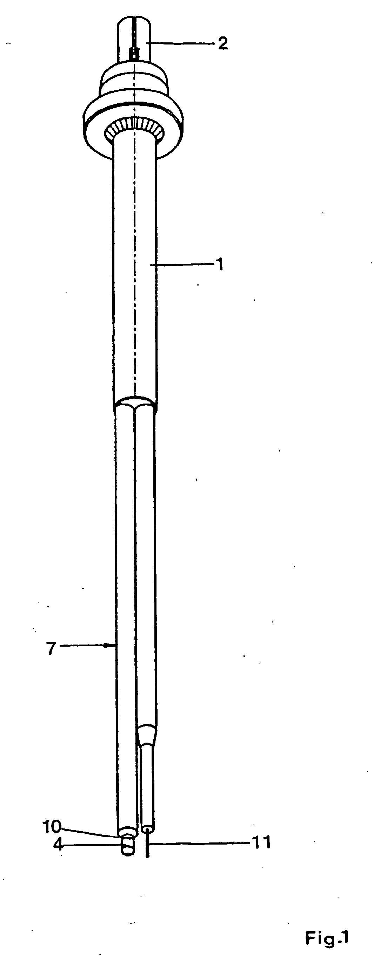

Die in Figur 1 dargestellte Vorrichtung zur Messung des Sauerstoffpartialdruckes weist ein Trägerrohr 1 auf, das aus Aluminiumoxid gebildet ist. An seinem dem Eintauchende abgewandten Ende ist ein Verbindungsstück 2 an dem Trägerrohr 1 angeordnet, das in einen in der Figur nicht dargestellten Halter, beispielsweise eine metallene Lanze eingesteckt wird. Die durch das Trägerrohr 1 hindurchgeführten Drähte, der Meßdraht 3 und die Referenzelektrode 4 werden über das Verbindungsstück 2 mit einem Meßsystem, das heißt mit einer üblichen Anzeige- und/oder Auswerteeinheit verbunden. Innerhalb des Trägerrohres 1 sind der Meßdraht 3 und die Referenzelektrode 4 durch Quarzröhrchen 5 geführt und in Kugelkorund 6 eingebettet.

An dem dem Eintauchende der Vorrichtung zugewandten Ende des Trägerrohres 1 sind die Referenzelektrodenanordnung 7 und das Quarzrohr 8 als hitzebeständiges Rohr angeordnet. Die Referenzelektrodenanordnung 7 weist ein Keramikrohr 9 aus Aluminiumoxid auf, durch das die Referenzelektrode 4 bis in das Festelektrolytröhrchen 10 hineingeführt ist. Das Festelektrolytröhrchen 10 aus Zirkonoxid weist in seinem Inneren eine Nickel-Nickeloxid-Pulvermischung auf, in der die Referenzelektrode 4, die aus einer Chrom-Nickel-Legierung (Cronix) gebildet ist, gehaltert ist. Durch das Quarzrohr 8 hindurch ist die Meßelektrode mit dem Meßdraht 3 geführt, die Spitze 11 der Meßelektrode ist aus Iridium-Draht gebildet. Sie kann jedoch auch aus einer Legierung, die überwiegend Iridium und darüber hinaus andere Edelmetalle enthält, gebildet sein. Die Spitze 11 aus Iridium ragt bis in das Quarzglasrohr 8 hinein. Die Spitze 11 ist in dem Ende 12 des Quarzglasrohres 8 gasdicht eingeschmolzen auf einer Strecke von etwa 2 cm. Danach wechselt das Material der Meßelektrode. Um den relativ teuren Iridium-Draht einzusparen, ist der Rest der Meßelektrode ein Meßdraht 3 aus Cronix (einer Chrom-Nickel-Legierung). Statt Cronix kann beispielsweise auch Molybdän oder Wolfram als Meßdraht 3 verwendet werden.

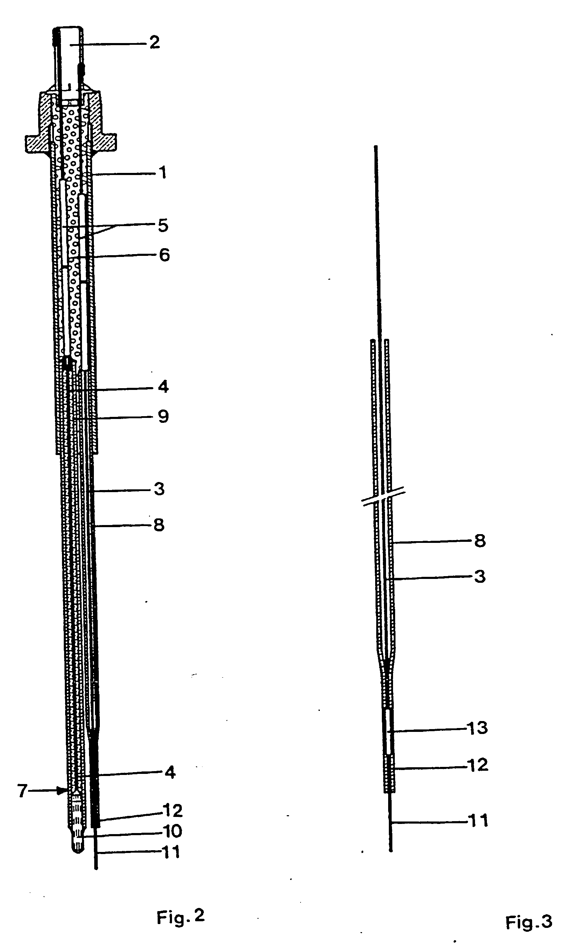

Eine weitere Möglichkeit, die Meßelektrode auszubilden, ist in Figur 3 dargestellt. Hier ist die Spitze 11 aus Iridium innerhalb des Quarzglasrohres 8 mit einem Molybdän-Streifen 13 verbunden, der an seinem anderen Ende mit dem Meßdraht 3 verbunden ist. Der Meßdraht 3 kann in diesem Fall beispielsweise aus Molybdän oder Wolfram gebildet sein. Der Molybdänstreifen 13 ist in dem gezeigten Beispiel vollständig in das Ende 12 des Quarzglasrohres 8 eingeschmolzen. Dadurch kann eine perfekte Gasdichtheit erzielt werden.

In den Figuren nicht dargestellt ist die Möglichkeit, den Molybdän-Streifen 13 aus dem zugeschmolzenen Ende 12 des Quarzglasrohres 8 herauszuführen und erst in dem offenen Rohr mit dem Meßdraht 3 zu verbinden. In einem solchen Fall wäre auch Cronix als Meßdraht 3 möglich.

Quarzglasrohr 8 und Keramikrohr 9 sind mit einer Korundfüllung versehen, die die Lage der Drähte innerhalb der Rohre stabilisiert.

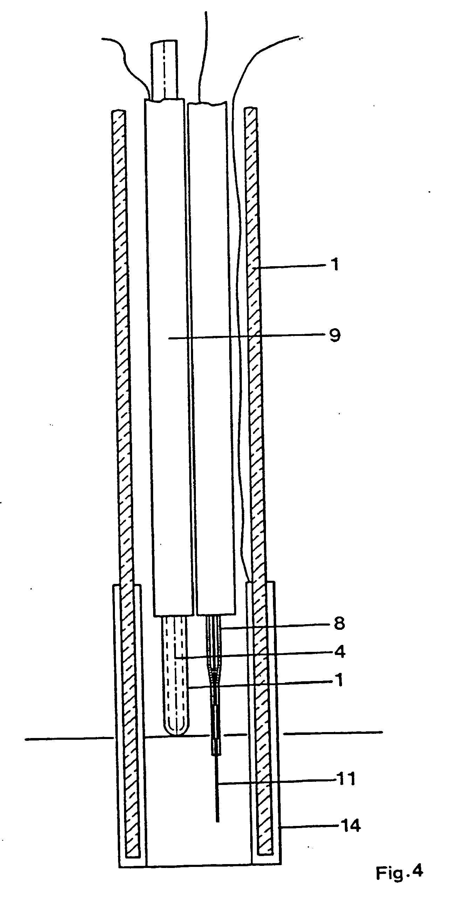

Die in Figur 4 dargestellte Vorrichtung ist zur voltametrischen Messung z. B. des Eisen-, Schwefel- oder Chromgehaltes in einer Glasschmelze geeignet. Das Verfahren hierzu ist beispielsweise in "Glastechnische Berichte" 68 (1995) No. 9, S. 273 ff. beschrieben. In dem Trägerrohr 1 aus Alumiumoxid sind eine Meßelektrode und eine Referenzelektrode 4 angeordnet. Die Spitze 11 der Meßelektrode ist aus Iridium gebildet und in ein Quarzglasrohr 8 eingeschmolzen. Die Referenzelektrode 4 aus Platin ist in einem Keramikrohr 9 angeordnet, und an dem Eintauchende des Trägerrohres 1 ist eine Gegenelektrode 14 aus Platin angeordnet.

Die Messung mit der beschriebenen Vorrichtung ermöglicht sehr zuverlässige Ergebnisse, in erster Linie im Kurzzeitbetrieb. Da die Vorrichtung sehr kostengünstig hergestellt werden kann, ist die Ausbildung als Einwegsonde möglich. Bei Verwendung eines hitzebeständigen Rohres 8 aus Aluminiumoxid ist auch ein Langzeiteinsatz denkbar.

## Patentansprüche

1. Vorrichtung zur Durchführung von elektrochemischen Messungen in Glas- oder Salzschmelzen mit wenigstens einer Meßelektrode und einer Referenzelektrodenanordnung, **dadurch gekennzeichnet, daß** die zum Eintauchen in die Schmelze bestimmte Spitze (11) der Meßelektrode aus Iridium oder einer Iridiumlegierung mit anderen Edelmetallen gebildet ist und in einem hitzebeständigen Rohr (8) gasdicht gehaltert ist, wobei die Meßelektrode durch das hitzebeständige Rohr (8) hindurchgeführt ist.

2. Vorrichtung nach Anspruch 1, **dadurch gekennzeichnet, daß** das hitzebeständige Rohr (8) ein Quarzglasrohr ist.

3. Vorrichtung nach Anspruch 1, **dadurch gekennzeichnet, daß** das Iridium oder die Iridiumlegierung in das hitzebeständige Rohr (8) gesintert oder eingeschmolzen ist.

4. Vorrichtung nach einem der Ansprüche 1 bis 3, **dadurch gekennzeichnet, daß** die Verbindung zwischen Meßelektrode und hitzebeständigem Rohr (8) an dem zum Eintauchen in die Schmelze bestimmten Ende (12) des Rohres (8) gasdicht ist.

5. Vorrichtung nach einem der Ansprüche 1 bis 4, **dadurch gekennzeichnet, daß** die aus Iridium gebildete Spitze (11) der Meßelektrode innerhalb des hitzebeständigen Rohres (8) mit einem Meßdraht (3) verbunden ist.

6. Vorrichtung nach Anspruch 5, **dadurch gekennzeichnet, daß** zwischen der aus Iridium gebildeten Spitze (11) der Meßelektrode und dem Meßdraht (3) ein Metallstreifen (13) aus Molybdän angeordnet ist.

7. Vorrichtung nach Anspruch 6, **dadurch gekennzeichnet, daß** der Meßdraht (3) aus Molybdän oder Wolfram gebildet ist.

8. Vorrichtung nach einem der Ansprüche 5 bis 7, **dadurch gekennzeichnet, daß** die Verbindung zwischen Iridium und dem Meßdraht (3) in dem hitzebeständigen Rohr (8) eingeschmolzen ist.

9. Vorrichtung nach Anspruch 5, **dadurch gekennzeichnet, daß** der Meßdraht (3) aus einer Chrom-Nickel-Legierung gebildet ist.

10. Vorrichtung nach einem der Ansprüche 1 bis 9, **dadurch gekennzeichnet, daß** eine Referenzelektrode (4) in einem einseitig geschlosenen Festelektrolytröhrchen angeordnet ist, das mit seinem dem geschlossenen Ende gegenüberliegenden Ende in einem Keramikrohr (9) gehaltert ist, durch das die Referenzelektrode (4) hindurchgeführt ist und daß das Ende der Referenzelektrode (4) in dem Festelektrolytröhrchen (10) von einem Referenzmaterial umgeben ist, das aus einer Metall-Metalloxid-, vorzugsweise einer Nickel-Nickeloxid-Pulvermischung gebildet ist.

11. Vorrichtung nach einem der Ansprüche 1 bis 10, **dadurch gekennzeichnet, daß** die Referenzelektrode (4) aus einer Chrom-Nickel-Legierung gebildet ist.

12. Vorrichtung nach Anspruch 10 oder 11, **dadurch gekennzeichnet, daß** das hitzebeständige Rohr (8) und das Keramikrohr (9) mit Korund gefüllt sind.

13. Vorrichtung nach einem der Ansprüche 10 bis 12, **dadurch gekennzeichnet, daß** das hitzebeständige Rohr (8) und und das Keramikrohr (9) in einem gemeinsamen Trägerrohr (1) gehaltert sind, das vorzugsweise aus Keramik gebildet ist und das an seinem dem Eintauchende abgewandten Ende ein Verbindungsstück (2) aufweist zur mechanischen Ankopplung und zur Verbindung von Meßelektrode und Referenzelektrode (4) mit einem Meßsystem.

14. Vorrichtung nach Anspruch 13, **dadurch gekennzeichnet, daß** das Trägerrohr (1) aus Aluminiumoxid gebildet ist und daß es mit Kugelkorund (6) gefüllt ist.

## Claims

1. An apparatus for taking electrochemical measurements in glass or salt melts, comprising at least one measuring electrode and one reference electrode arrangement, **characterised in that** the tip (11) of the measuring electrode that is intended to be immersed in the melt is formed from iridium or an iridium alloy comprising other noble metals and is held in a heat-resistant tube (8) in a gastight manner, wherein the measuring electrode is passed through the heat-resistant tube (8).

2. The apparatus according to Claim 1, **characterised in that** the heat-resistant tube (8) is a quartz glass tube.

3. The apparatus according to Claim 1, **characterised in that** the iridium or the iridium alloy is sintered or fused into the heat-resistant tube (8).

4. The apparatus according to anyone of Claims 1 to 3, **characterised in that** the connection between the measuring electrode and the heat-resistant tube (8) is gastight at that end (12) of the tube (8) that is intended to be immersed in the melt.

5. The apparatus according to anyone of Claims 1 to 4, **characterised in that** the tip (11) of the measuring electrode that is formed from iridium is connected to a measuring wire (3) inside the heat-resistant tube (8).

6. The apparatus according to Claim 5, **characterised in that** a metal strip (13) made of molybdenum is arranged between the tip (11) of the measuring electrode that is formed from iridium and the measuring wire (3).

7. The apparatus according to Claim 6, **characterised in that** the measuring wire (3) is formed from molybdenum or tungsten.

8. The apparatus according to anyone of Claims 5 to 7, **characterised in that** the connection between the iridium and the measuring wire (3) is fused into the heat-resistant tube (8).

9. The apparatus according to Claim 5, **characterised in that** the measuring wire (3) is formed from a chrome nickel alloy.

10. The apparatus according to anyone of Claims 1 to 9, **characterised in that** a reference electrode (4) is arranged in a solid electrolyte tube which is closed on one side and which, with its end disposed opposite to the closed end, is held in a ceramic tube (9) through which the reference electrode (4) is passed, and that the end of the reference electrode (4) in the solid electrolyte tube (10) is surrounded by a reference material which is formed from a powder mixture comprising a metal and metal oxide, preferrably from a powder mixture comprising nickel and nickel oxide.

11. The apparatus according to anyone of Claims 1 to 10, **characterised in that** the reference electrode (4) is formed from a chrome nickel alloy.

12. The apparatus according to Claim 10 or 11, **characterised in that** the heat-resistant tube (8) and the ceramic tube (9) are filled with corundum.

13. The apparatus according to anyone of Claims 10 to 12, **characterised in that** the heat-resistant tube (8) and the ceramic tube (9) are held in a common carrier tube (1) which is, preferrably, formed from ceramic and, at its end facing away from the immersion end, comprises a connecting piece (2) for mechanical coupling and for connecting the measuring electrode and the reference electrode (4) to a measuring system.

14. The apparatus according to Claim 13, **characterised in that** the carrier tube (1) is formed from aluminium oxide and that it is filled with spherical corundum (6).

## Revendications

1. Dispositif pour effectuer des mesures électrochimiques dans des bains de verre en fusion ou des bains de sels fondus, comprenant au moins une électrode de mesure et un agencement d'électrode de référence, **caractérisé en ce que** la pointe (11) de l'électrode de mesure, qui est destinée à être immergée dans le bain de fusion, est réalisée en iridium ou en un alliage d'iridium avec d'autres métaux nobles, et est supportée de manière étanche aux gaz dans un tube réfractaire (8), l'électrode de mesure étant menée à travers le tube réfractaire (8).

2. Dispositif selon la revendication 1, **caractérisé en ce que** le tube réfractaire (8) est un tube de verre quartzeux.

3. Dispositif selon la revendication 1, **caractérisé en ce que** l'iridium ou l'alliage d'iridium est noyé par frittage ou par fusion dans le tube réfractaire (8).

4. Dispositif selon l'une des revendications 1 à 3, **caractérisé en ce que** la liaison entre l'électrode de mesure et le tube réfractaire (8) est étanche aux gaz à l'extrémité (12) du tube (8), qui est destinée à être immergée dans le bain de fusion.

5. Dispositif selon l'une des revendications 1 à 4, **caractérisé en ce que** la pointe (11) en iridium de l'électrode de mesure est reliée, à l'intérieur du tube (8) réfractaire, à un fil métallique de mesure (3).

6. Dispositif selon la revendication 5, **caractérisé en ce qu'**entre la pointe (11) en iridium de l'électrode de mesure et le fil métallique de mesure (3) est agencé une bande de métal (13) en molybdène.

7. Dispositif selon la revendication 6, **caractérisé en ce que** le fil métallique de mesure (3) est formé de molybdène ou de tungstène.

8. Dispositif selon l'une des revendications 5 à 7, **caractérisé en ce que** la liaison entre l'iridium et le fil métallique de mesure (3) est noyée par fusion dans le tube réfractaire (8).

9. Dispositif selon la revendication 5, **caractérisé en ce que** le fil métallique de mesure (3) est réalisé en un alliage de nickel-chrome.

10. Dispositif selon l'une des revendications 1 à 9, **caractérisé en ce qu'**une électrode de référence (4) est agencée dans un petit tube d'électrolyte solide fermé d'un côté, qui avec son extrémité opposée à l'extrémité fermée est supporté dans un tube de céramique (9) à travers lequel est menée l'électrode de référence (4), et **en ce que** l'extrémité de l'électrode de référence (4) dans le tube d'électrolyte solide (10) est entourée par un matériau de référence, qui est formé par un mélange de poudre de métal - oxyde de métal, de préférence de nickel - oxyde de nickel.

11. Dispositif selon l'une des revendications 1 à 10, **caractérisé en ce que** l'électrode de référence (4) est réalisée en un alliage de nickel-chrome.

12. Dispositif selon la revendication 10 ou 11, **caractérisé en ce que** le tube réfractaire (8) et le tube de céramique (9) sont remplis de corindon.

13. Dispositif selon l'une des revendications 10 à 12, **caractérisé en ce que** le tube réfractaire (8) et le tube de céramique (9) sont supportés dans un tube de support (1) commun, qui de préférence est réalisé en céramique, et qui présente à son extrémité opposée à l'extrémité d'immersion, une pièce de liaison (2) pour le couplage mécanique et pour la liaison de l'électrode de mesure et de l'électrode de référence (4) avec un système de mesure.

14. Dispositif selon la revendication 13, **caractérisé en ce que** le tube de support (1) est réalisé en oxyde d'aluminium et **en ce qu'**il est rempli de corindon en billes (6).
